# EUROPEAN PATENT SPECIFICATION

(11) **EP 3 685 796 B1**
(45) Date of publication and mention of the grant of the patent: **17.04.2024**
(21) Application number: 19153325.6
(22) Date of filing: 23.01.2019
(51) Int. Cl.: A61C 9/00, A61C 13/00, A61C 13/08, G01J 3/50, G06T 7/90, A61C 5/77, A61C 5/20, A61C 19/10, A61C 13/083

(54) **COMPUTER IMPLEMENTED METHOD FOR CUSTOMIZED COLORATION OF DENTAL RESTORATIONS, DEVICE THEREOF, AND DENTAL RESTORATION**
COMPUTERIMPLEMENTIERTES VERFAHREN ZUR INDIVIDUELLEN FÄRBUNG VON ZAHNRESTAURATIONEN, VORRICHTUNG DAFÜR UND ZAHNRESTAURATION
PROCÉDÉ MIS EN OEUVRE PAR ORDINATEUR POUR LA COLORATION PERSONNALISÉE DE RESTAURATIONS DENTAIRES, DISPOSITIF À CET EFFET ET RESTAURATION DENTAIRE

(43) Date of publication of application: 29.07.2020
(73) Proprietor: Sirona Dental Systems GmbH, 64625 Bensheim (DE); DENTSPLY SIRONA Inc., York, PA 17401 (US)
(72) Inventor: FRANKE, Frederike, 64625 Bensheim (DE); BRANDES, Dr. Christoph, 64625 Bensheim (DE); BÄURER, Michael, 64625 Bensheim (DE); VOß, Björn, 64625 Bensheim (DE); KUCHARCZYK, Ronny, 64625 Bensheim (DE)
(74) Representative: Özer, Alpdeniz

(56) References cited:
- WO-A1-2013/095968
- WO-A1-2013/122662
- US-A1- 2010 303 315
- US-A1- 2012 205 828
- US-B1- 10 007 987

## Description

### Field of the invention

The invention relates to a system comprising a computer program for designing or virtually preparing color customization of dental restorations.

### Background and general description of the invention

Dental restorations are applied to restore the natural appearance of the oral cavity in the event of tooth damage or tooth loss. In addition, a dental restoration becomes part of the chewing apparatus and is thus exposed to abrasion by chewing and grinding processes. It is known how to make dental restorations resistant and adapted in shape to the gap to be filled.

A color adaptation of dental prostheses to the individual intraoral situation of a patient, in which the corresponding dental prosthesis is to be used, is also known in dentistry. Typically, a standardized tooth shade key (e.g., VITA classical A1-D4^{®} shade guide) is used to determine the shade of the restoration. Materials for the production of dental restorations are usually offered in different basic colors (tooth colors). There are also materials for the production of dental restorations, in which a color gradient is already realized in the material in order to represent the intraoral color gradient closer to reality. But even this color adaptation does not always correspond to the color that fits the individual aesthetic of the oral situation and which should have been used. It is known how to stain the prosthesis typically manually with a color according to the tooth shade key, thereby improving the result. Especially in the field of teeth and dental prostheses, aesthetics play an extraordinary role. It may even be the case that patients sometimes only accept dental prostheses that are difficult or impossible to recognize as such.

Customized staining a dental prosthesis to obtain the most authentic replica or copy of a natural tooth that is color-matched to a patient's intraoral situation is a challenging task that, if desired, is typically done by specialists with many years of experience in a dental laboratory in a handcrafting manner. This leads to a high demand for corresponding specialists and, correspondingly, to high costs for such customization.

Reference is made to US 2012/0205828A1 which discloses a method for producing a dental restoration part and Cad/Cam device. Further reference is made to WO 2013/095968A1 which discloses a method and system for making a dental restoration.

The inventors have recognized that a dental restoration that can be highly customized to the individual situation while remaining cost effective has a significant sales advantage. The object of the present invention is to provide a system comprising a computer program provided with instructions for designing or virtually preparing color customization of dental restorations, which is simple to use and already due to this considerably reduces the costs, so that said instructions can be performed by a large group of people and that the implementation is not limited to a few specialists.

The present invention therefore allows for a dentist, who produces a restoration by means of a digital impression, to be able to color customize the same. For example, restorations that were previously sent to a dental laboratory because of the complexity of the color can now be produced on-site and therefore also spare the patient from having to have further sessions, where applicable. A further disclosure, which does not form part of the claimed invention, is to provide an especially cost-effective but nevertheless high-quality method for the color customization of dental restorations. An embodiment of the present invention also relates to a device for color customization, by means of which a dental restoration can be stained.

By means of the invention the disadvantages mentioned in the description in relation to known systems may be overcome.

The above objectives of the present invention are achieved by the method as defined in claim 1. The dependent claims relate to further developments and embodiments.

The invention provides a system comprising a computer program for designing or virtually preparing color customization of dental restorations. The computer program comprises instructions which involve a step "providing tooth measurement data" in which information from tooth measurement data is provided. This information is typically 3D data plus color information covering at least the area of application in which the dental restoration is to be inserted. In other words, for example, with a computing unit such as a computer, a previously created record of tooth measurement data is imported. The data set of the tooth measurement can already be stored on the computing unit. However, it is also disclosed that the tooth measurement data are first created with this or an associated computing unit and is made available directly to the method for color customization, which does not form part of the claimed invention. In this case, "providing" means taking over the data of the tooth measurement by the program structure, which carries out the calculations to be carried out at the beginning in the context of the method for color customization of dental restorations.

The computer program instructions further includes the step of "providing restoration data". In other words, the aforementioned program structure acquires data describing the dental restoration, for example with regard to the material structure or a material base color. For example, the data may be stored in a table and retrieved from the program structure as part of the method. The restoration data may also be entered as a manual input by a user during the course of the procedure. In this variation, the acquisition of the manual input of restoration data by the program structure represents the step "providing restoration data" through the program structure.

Further, the computer program instructions comprises the step of "providing staining or glazing color data". For example, stains are used when the restoration is sintered after staining, so that the colors burn in. Glazing colors are used when the restoration is only glazed after staining.

For example, when reading in the staining or glazing colors, the program structure acquires data from a color table in which the characteristics that specify the colors are listed, and which characteristics can be used to color customize the restoration. Such characteristic data can contain, for example, how the corresponding colors interact with the material of the restoration to be used, the composition of the respective color or which shade of color the respective color has. Intensity or color can vary on the material of the restoration to be used. For example, dental ceramics can be used as the material of the restoration.

According to the invention, a color characteristic is generated from the tooth measurement data or other information taking into account the restoration data. In one example, the color characteristic comprises a grid, in particular a 3d grid, of the determined tooth surface or, as the case may be, the surface of the restoration, wherein a color information is assigned to each grid point of the grid. The grid points of the color characteristic can be analyzed with regard to the assigned color value. In other words, grid values of a grid are assigned color values on the basis of the data of the tooth measurement data. The grid can also be in the form of a triangle mesh. Other information may be used if the dental measurement data is not suited to generate a color characteristic due to functional or aesthetic reasons.

In another example, the color characteristic comprises pixels of a color value image. Said pixels are preferably assigned to different predefined areas, such as an insertion area or a comparison area.

Subsequently, for example by means of a computing unit such as a personal computer, a multicolor target coloration is calculated from the color characteristic taking into account the color data. The target coloration includes, for example, the information on whether a grid point of the color characteristic should be assigned to a certain color or should be left untreated. For example, it is possible to set a threshold value according to which a color value identical or similar to the basic color of the restoration is not colored. In an analogous manner, thresholds for color values can be assigned to the individual colors, so that one color is uniformly used within a region of color tones. An algorithm can calculate the appropriate color mapping. In one example, the algorithm can perform color interpolations between the color points or grid points and thus smooth the color gradients. The desired or target coloration indicates which color point or grid point of the three-dimensional lattice receives a color value change, i.e. a color customization. For this purpose, information can be acquired and processed for the calculation of the target coloration, taking into account, for example, the colors to be used, their application widths and their interaction with the blank material of the restoration, that means the material from which the restoration will be produced. Thus, the target color can be used as a guide at which points or grid points or in which areas of the restoration which colors are to be used and, for example, which stroke widths, color intensities or hues are each beneficial. For example, color intensities and shades can be regulated based on the applied amount. Different color types can be provided for different tooth types. For example, color types can be provided for the customization of incisal areas, or those for fissures on molar teeth. Furthermore, color types can be provided with which the basic color of the restoration may be changed in a particular color direction.

The multicolor target coloration for the color customization of the dental restoration is output. An example of an output of the multicolor target coloration is the output of the aforementioned data of color usage, stroke width, color intensity, density of inking on a display device such as a computer screen; The aforementioned data may be illustrated superposed with an image of the restoration, so that a representation of the use of colors is clearly visible. It could be said that a color template is visualized on the display device, which the user uses to color the restoration. However, this is just one example of several how the calculated target color can be output for further use.

The tooth measurement data are available preferably in the form of a graphic color image. For example, it is recorded with a 3D camera. From the camera image, a surface grid of the tooth situation can be generated. The surface grid can be divided into areas, for example the insertion area, so that grid points with their color values can be assigned to the insertion area.

The tooth measurement data may further comprise a comparison area, wherein said comparison area is arranged, for example, adjacent and / or opposite to the insertion area. The comparison area can also be recorded before the preparation of the tooth or teeth to be treated in order to record the color information of the tooth substance removed later. In other words, teeth adjacent to the area to be filled are determined, that is, for example, teeth adjacent to the tooth gap, the tooth gap representing the insertion area, and these teeth are associated with said comparison area. Thus, the color values of the grid points associated with an adjacent tooth are assigned to the comparison area.

The target coloration can be determined from the insertion area and the comparison area. For example, a multi-dimensional linear interpolation of the color values in the color characteristic can be used to produce homogeneous color transitions between the comparison area or region and the insertion area or region.

In a preferred embodiment, the step "providing tooth measurement data" can also include the importing of tooth measurement data of an unprepared tooth and the importing of second tooth measurement data of a prepared tooth, wherein the prepared tooth is prepared for the subsequent treatment. In other words, a first 3D image of the intraoral situation can be created, wherein the insertion area is unprepared, and a second 3D image of the oral situation is created, wherein the insertion area has already been prepared for the reception of the dental restoration.

Before the step "providing tooth measurement data", the step "creating tooth measurement data" can be included, in particular as a colored 3D image of the individual intraoral dental situation. In other words, at first tooth measurement data of an intraoral tooth situation are generated with a suitable recording device and are then imported by the program structure for further processing for generating the color characteristic.

In order to generate the color characteristic from the tooth measurement data, regions can advantageously be identified in the tooth measurement, in particular the insertion area, and a subdivision of the regions, such as tooth structure regions, can be carried out. Tooth structure regions can be assigned to specific tooth regions, such as tooth flanks or fissures. If the tooth measurement has pixels and each pixel has an image color value, or if grid points are calculated, the image color values or grid point color values may be assigned to the respective region of the tooth measurement.

A model color scheme in the form of a color distribution map can be stored in the program structure, which contains a standard proposal for each tooth type, which is then adapted to the individual situation on the basis of the color information from the insertion area and the comparison area. This may provide already a useful result or may be the basis for further calculation.

Alternatively, the color characteristic can also be freely designed by the user without reference to dental measurement data by gaining other information (f. e. by "Smile Design"). This may be of particular importance if the comparative data or the existing residual tooth substance are unsuitable for functional and / or aesthetic reasons.

Calculating a target coloration further includes applying an algorithm to determine a desired or target color characteristic of the restoration. The algorithm takes into account the restoration data, recorded colorimetric data, 3D surface data and information on staining or glazing colors.

The computation of a target coloration also includes the generation of colors to be used, color gradients and / or color line widths.

The step of "calculating a target coloration" includes reading in comparison data, applying an algorithm performing a comparison of the comparison data with the color characteristic, and wherein the algorithm performs a transfer step in which the target coloration is generated from the data of the color characteristic, for example considering tooth structures. In other words, the structural data of the color characteristic and comparison data are determined in advance and it is determined how far the color characteristic coincides with the comparison data or deviates therefrom. Examples of the inclusion of comparative data are color values from earlier color photographs or from laboratory experiments; however, comparison data can also be determined from a comparison area.

The step of "calculating a target coloration" includes determining color gradients that are producible or derivable from the color characteristic, as well as generating the target color characteristic using the color gradients.

The comparative data are be obtained from dental measurement data and target colorations of previous dental restorations. Alternatively or additionally, the comparative data can be received from data obtained in laboratory experiments.

The algorithm is prepared to calculate color gradients from the color characteristic, wherein, for example, mathematical interpolation means are being used for calculating the color gradients. The algorithm may also include an artificial intelligence to evaluate the color situation of the tooth measurement data and, therefrom, to calculate the color characteristic.

The step of "outputting the target coloration" may further include displaying the target coloration on a display device. Alternatively or additionally, the colors, color gradients and / or color line widths to be used can be displayed on the display device. Furthermore, the output step may include outputting the target coloration on a printer, and / or outputting the target coloration on a data carrier.

The restoration data contains at least one of the following information: a blank material or a blank base color.

Furthermore, the step "providing restoration data" comprises scanning the restoration material, in particular after a pre-sintering step.

The dental restoration may be prepared to close a single tooth gap or to close multiple gaps.

The step "output of the target coloration" may further include the color customization of the dental restoration with different staining or glazing colors, such as "Coloring Liquids" (aqueous solution of metal ions for sintered ceramics) in such a way that the dental restoration receives the target coloration.

After the step "output of the target coloration", the step "finalizing of the dental restoration" can take place. Finalizing can be for example re-sintering, crystallization or glazing.

Furthermore, the producing a colored dental restoration is covered by the invention, comprising the steps of "creating tooth measurement data", for example as a colored 3D image of an intraoral dental situation, wherein the tooth measurement data comprise at least one insertion area into which the dental restoration is to be inserted; "providing restoration data" for determining at least the blank material and the blank base color; "providing information about staining or glazing colors"; "generating a color characteristic" from the tooth measurement data taking into account the restoration data; "calculating a multicolor target coloration" from the color characteristic taking into account the information about staining or glazing colors; "manufacturing of the dental restoration" from a blank material and, "output of the target coloration" for applying the target coloration on the restoration; and, optionally, sintering or crystallizing the same.

The disclosure also includes a dental restoration produced by the method as described above.

Further, a device for color customization of dental restorations is disclosed, wherein the device comprises a computing unit with a memory for depositing tooth measurement data, the tooth measurement data comprising at least one insertion area, in which the dental restoration is to be inserted, and further for storing restoration data and information about staining or glazing colors. The device further comprises a processor for generating a color characteristic from the tooth measurement data taking into account the restoration data, and for calculating a target coloration from the color characteristic, taking into account the information on staining or glazing colors. The device further comprises an output device for visualizing the multicolor target coloration for color customization of the dental restoration.

In a preferred embodiment, the device for color customization of dental restorations may comprise a display device for displaying the target coloration, and / or a printer for outputting the target coloration, and / or a writing device for outputting the target coloration on a data carrier.

Furthermore, the device may comprise a coloration device, which is adapted to apply the multicolored target coloration on the dental restoration.

The coloration device can provide a plurality of different coloring solutions. The coloration device is preferably adapted to automatically apply the colors to the dental restoration.

The coloration device may preferably provide multiple (for example 9) or more different coloring solutions. More preferably, the coloration device is designed to provide the coloring solutions in different line widths.

The device may comprise an optical recording device for checking the application of color to the dental restoration. This can be performed, for example, with the recording system with which the tooth measurement data has previously been performed.

Thus, applying the colors can be continuously monitored, in the manner of a feedback system, and in the event of a deviation from the target coloration, a signal, for example acoustically or optically, can be emitted or the coloration can be automatically corrected.

### Brief description of the figures

It is shown in:
- Fig. 1: Flowchart of the color customization procedure,
- Fig. 2: example of an output generated by the method,
- Fig. 3: a first example of a coloration device,
- Fig. 4: another example of a coloration device.

### Detailed description of the invention

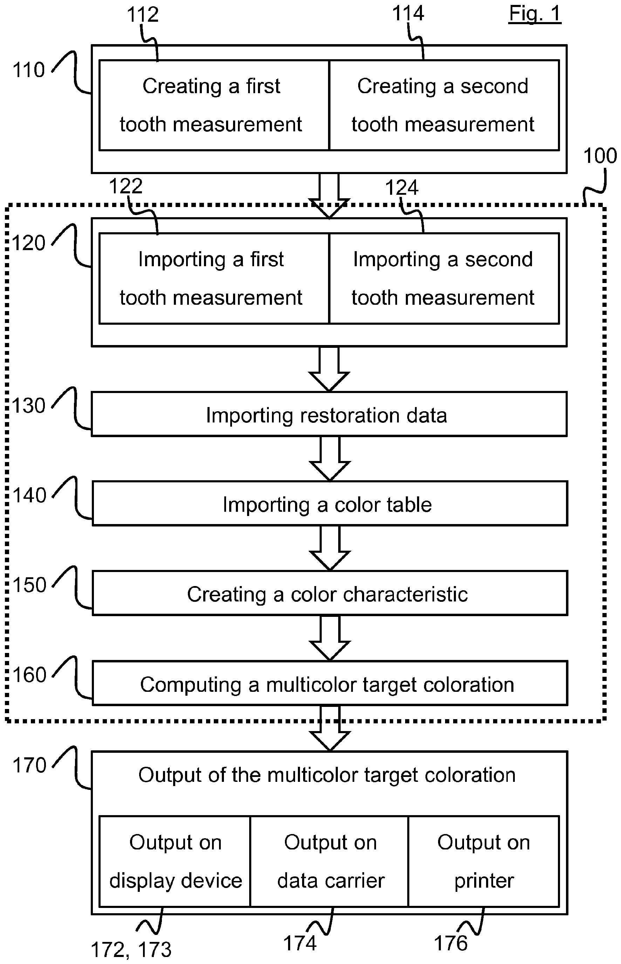

Referring to Figure 1, there is shown a flow chart for the color customization method executable by the system according to the invention, with preferred intermediate steps. In a tooth measurement step 110, tooth measurement data are created. For example, the basis of a tooth measurement can be a 3D image of a patient's oral situation created e.g. with an Omnicam^{®} camera.

Optionally, a first dental measurement 112 and a second dental measurement 114 can be made. This may be the case if first the initial insertion area with the original oral situation with a defective tooth is recorded for a first tooth measurement 112, then, for example for a second tooth measurement 114, the already prepared insertion area is recorded in which the preparations for the reception of the dental reconstruction are completed.

In the tooth measurement data providing step 120 the created tooth measurement data are imported. The import can be performed by a program structure 100. The program structure 100 may perform, for example, all the steps 120, 130, 140, 150, 160 associated with the computer-based computation of the data until outputting a target coloration. It may also be provided that the tooth measurement data creation step 110 is performed by the same program structure 100. Then, if necessary, the tooth measurement data providing step 120 can be dispensed with or, as the case may be, it may then be the passing on the data of the tooth measurement within the program structure 100. If two tooth measurement data 112, 114 have been created, the importing of the tooth measurement data can also take place in the steps 122, 124.

In a restoration data providing step 130, data about the restoration, for example the restoration material and the restoration base color, are loaded into the program structure 100. The import of the restoration data 130 may optionally be performed manually by a user, or it may be carried out in time prior to the tooth measurement 110. In any case, by means of the restoration data providing step 130 the program structure 100 has access to the restoration data and they can be taken into account for further calculations.

In an importing step 140 of information on staining or glazing colors, data on color types are imported like staining or glazing colors, color shades, color values and / or color intensities, the color effect on different materials, but also color availability, manufacturer codes, etc. can be stored.

In a color characteristic generation step 150, the imported data 120, 130, 140 are merged and a color characteristic is generated for the restoration. For example, from the tooth measurement data 120 surface structures are recognized and assigned to areas or regions. By way of example, such a region may be the insertion area or the comparison area.

The surface structure may be present as a grid, as a mesh, as a 3d grid, etc. The measured surface can also be represented as a triangle mesh. To each triangle, or to each node in the network, a color information of the associated surface portion of the tooth surface or the surface of the reconstruction can be assigned. The color values of the grid points can be extracted and assigned to areas or regions. The restoration data can then be assigned to the individual areas, such as the restoration base color.

From the merged information, in a target coloration calculation step 160 finally a multicolor target coloration is calculated. Color preferences can already be assigned by means of the region or structure information. For example, rather areal customization are preferred on buccal surfaces or side surfaces of teeth; in the vicinity of fissures or edges, such as the incisal edge of the incisors, fine structures are preferred. On medium-sized structures such as e.g. in distal dimples or on cusps of molar teeth, in turn, average customization are preferred. This has an influence on the choice of coloring suggestions, for example with regard to the stroke widths to be used. In this case, a suggestion color distribution map can be stored in the program structure 100 for different types of teeth, which are successively approximated to the individual oral situation of the insertion area on the basis of the color information of the insertion area and advantageously also of a comparison area such as a neighboring tooth or other teeth.

The selected restoration material has an influence on the colors to be used, since there may be interactions between the colors and the material, which results in a different effect of the color. In the target coloration calculation step 160, color gradients may be obtained from the data of the color characteristic 150, and color unevenesses such as color hue or local color deviation may be detected.

The target color gradients for the restoration can now be determined from this by means of a mathematical model. By way of example, this can be done separately for each determined region of the restoration, wherein the region separating lines are assigned to known structure separation lines such as a fissure. An example of a mathematical model is a multidimensional linear regression over the color values assigned to a region.

Another example for calculating the target color gradients is the use of an artificial intelligence programmed and set up for this purpose, which, for example, is designed to independently learn on the basis of a test data set to adapt color distributions to reconstructions. It may use color gradient data from other teeth, which is, for example, from comparison areas comprising adjacent teeth and / or teeth in the opposite quadrant. The artificial intelligence can be trained with example color gradients of laboratory-generated reconstructions and this information can be used in the creation of new color gradients of reconstructions to be manufactured.

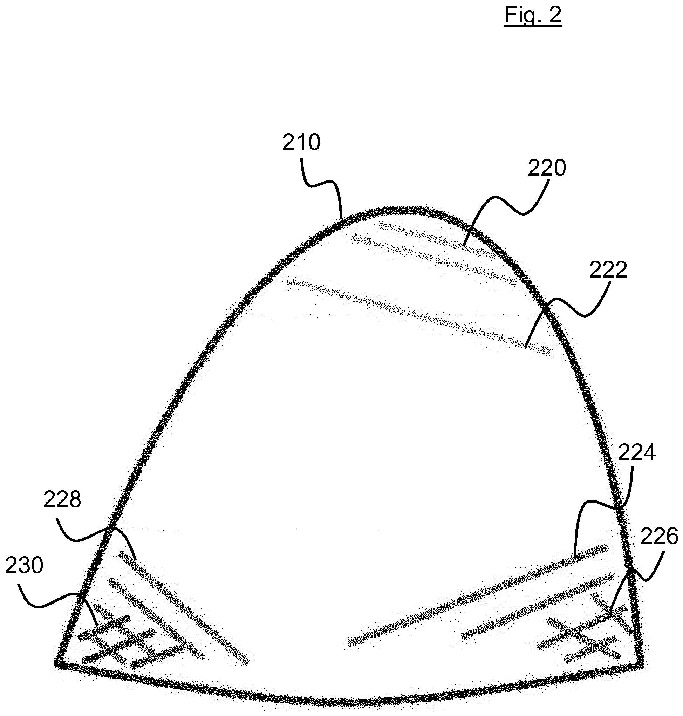

In addition to the determined color information for the color gradients to be achieved on the dental restoration, it is determined which region or area is to be colored with a specific color type and a specific color intensity in order to calculate the target coloration 160. In other words, it is determined, which of the available colors is to be applied in which available line width and possible intensity to which areas of the restoration, see also Fig. 2.

For determining the target coloration 160 to be achieved, the color difference between the base color of the restoration material and the target color can first be determined at each grid point or triangle point. Thereafter, in the calculation of the target coloration 160, it can be identified which of the available color and / or pen types optimally reduces this color difference. A possible output 170 is in which region of the restoration a particular color type and / or pen type is to be applied. Advantageously, a threshold value for color differences can also be provided, so that with little deviation from a base color or a type of color or type of pen to be used no change of color type or pen type is performed, but e.g. a surface is stained uniformly.

Finally, in an output step 170, the calculated target coloration 160 is output. For example, the output 170 is output on a display device 172, such as a computer screen. In this case, it is possible to provide a user with instructions for the colors to be applied on the restoration, by displaying, for example, colors, pens and stroke widths on the display 172. In another example, the output may be on a data carrier 174 for storing the calculated target coloration 160, for example, for delivery to a dental laboratory. In yet another example, the output may be performed on a printer 176, for example a foil printer. Such a foil printer can print on a tooth foil, which is advantageously applied to the surface of the restoration for a subsequent sintering step or glaze firing, so that coloring of the restoration takes place during the re-sintering or the glaze firing. Said foil is preferably made of organic material which burns residue-free during the sintering process.

Fig. 2 shows a sketch of a possible output of a target coloration 160 on a dental restoration 210. In this example a restoration of an incisor tooth, having for simplicity of illustration only one buccal side as a structural region, is shown. A first color type "A" is applied 220 at the top of the structure area in a first density. The application density of the color is illustrated by the spacing of the lines 220. The same color type "A" is continued with a lower application density 222 towards the center of the structure area. In the middlemost region of the structure, in the example of Fig. 2 shown here, no customized color application is required, since the basic color of the restoration material matches already sufficiently well the target tooth color.

In a right side portion of the dental restoration 210 in Fig. 2, representing a multicolor target coloration 160, a second color type "B" is applied. The direction and density 224, 226 of the color application of color type "B" are included in the output of the target coloration 160. Thus, initially a nearly horizontal first lining 224 with a first line density is applied, followed by a second lining 226 with a second line density to be applied at a displayed angle.

In a left side portion of the dental restoration 210 in Fig. 2, another color application 228 of the color type "B" is applied. The target coloration 160 shows in the output 170 the color application 228 with a pre-calculated line density. For example, tooth customization colors can be applied by a user directly to the restoration 210. In doing so, he can use the computation of the target coloration 160 because the output 170 directs the user to which colors and line widths to apply on the restauration. In the same left side portion of the dental restoration 210, a stain job 230 with the color type "C" is applied on top of the previous stain job 228. This can result in a mixed color. The stain job 230 with the color type "C" is also specified with regard to the line density. Further, the stroke width of the job 230 is, for example, wider than the stroke width of the stain job 220. This may indicate the application of a wider pen, a wider brush or a wider region spray of the stain with a spray head or similar stain application.

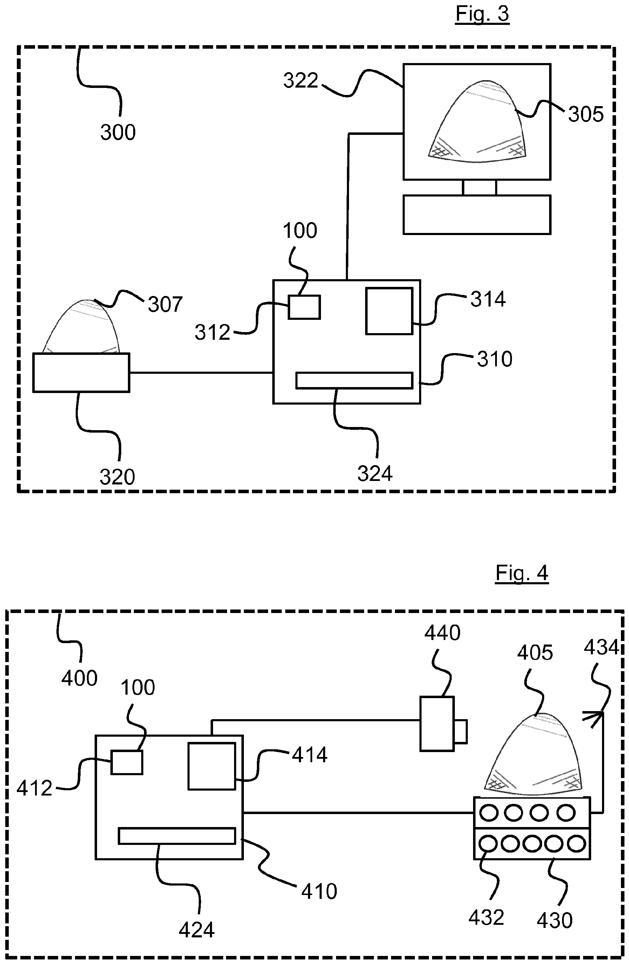

Referring to Fig. 3, an embodiment of a device 300 for color customization of dental restorations is shown. A computing unit 310 accommodates a memory 312 in which the program structure 100 is stored, and a processor 314 for processing the processing steps of the program structure 100. Tooth measurement data 110, 112, 114 can be stored in the memory 312, wherein the tooth measurement data comprise at least one insertion area into which the dental restoration is to be inserted. Furthermore, restoration data 130 and the corresponding information about staining or glazing colors 140 may be stored in the memory 312. By means of the processor 314 and by considering the aforementioned data, the color characteristic 150 and herefrom the multicolor target coloration 160 can be calculated, which can be output, for example, as a target template display 305 or as a color application foil 307.

The device 300 shown in Fig. 3 has in its embodiment three output devices 320, 322, 324. First, a printer 320 is illustrated by means of which the film or foil 307 can be dispensed. The color application foil 307, which is printed with the printer 320 with the colors to be applied, can be applied to the dental restoration and sintered again with the dental restoration. The foil is designed in such a way that the coloring elements are transferred to the restoration during the sintering process and that the transport foil carrying the coloring elements burns without residue.

A display device 322 is connected to the computing unit 310. The display device 322 shows a coloring template 305, by means of which a user can stain a dental restoration in a particularly simple and intuitive manner.

A writing unit 324 is set up to write a data carrier with the target coloration 160 calculated by the computing unit 310.

The output devices 320, 322, 324 can be operated independently. Thus, in a similar example, the computing unit 310 may also be merely connected to the display device 322, or may comprise only the printer 320 etc.

Referring to Fig. 4a further device 400 for assisting dental experts during the color customization of dental restorations 405 is illustrated. A computing unit 410 comprises a memory 412, for storing the program structure 100, and further a processor 414 for performing the computations with the program structure 100. The computing unit 410 further comprises a write unit 424 for writing the target coloration 160 on a data carrier.

In the embodiment of Fig. 4, an automated stain device 430 is provided. The automated stain device 430, in the illustrated embodiment, comprises multiple (for example 9) different colors 432 that can be set and used individually by the stain device 430 in response to the instruction of the computing unit 410 according to the target coloration 160. Depending on the application, they may be staining colors 432, or they may be glazing colors 432.

It has been shown in various experiments that a storage with nine colors allow sufficient customization types for dental restorations required by dental professionals. However, it is not excluded that a stain device 430 - or a user - uses more or less than nine colors 432, for example 8 colors, 7 or 6 colors, or else 10, 11 or more colors, in order to obtain the best possible customization result of the dental restoration. More preferably, more than 6 colors 432 can be used.

For example, the stain device 430 may have a color receptacle 436 that accommodates 4 to 30 different color types, preferably 7 to 30, particularly preferably 9 to 30 or 9 to 20 color types. Cumulatively or alternatively, the stain device 430 may include various spray heads 434 that stain wider or narrower areas and, if necessary, apply stronger or weaker intensities.

In an analogous manner, the output of the target coloration 160 on the display device 322 may represent a distinction of 4 to 30 different color types and / or output different line widths. Preferably, it may be 7 to 30, more preferably 9 to 30 or 9 to 20 different color types, which are displayed differently on the display device 322, so as to guide the user to use the appropriate color type and / or the corresponding stroke width.

The automated stain device 430 has a spray unit 434, by means of which the dental restoration 405 can be sprayed in accordance with the target coloration 160 with the different colors and possibly different line densities.

The staining or glaze dyes 432 have a staining solution which comprises, for example, metal salts based on chlorides, for example MnCl₂, TbCl₃, ErCl₃, PrCl₃ NdCl₃ or CrCl₃. It is also possible to use for example staining solutions that comprise metal salts based on acetates. The color solution is tested for the various, for example, ceramic materials of the restoration material to determine mixing effects with the material and resulting color changes.

The device 400 further may comprise an optical recorder 440 for recording the staining process on the dental restoration 405. Should there be deviations from the target coloration 160 in the stain application by the spray head 434, for example, triggered by a clogged nozzle of the spray head 434 or other influences, the computing unit 410 may respond to the deviation and counteract either with a changed color application, undo the inking or cancel the stain job. In other words, the device 400 may comprise a feedback system in which a camera 440 monitors the stain application by the automated stain device 430 and reports deviations to the computing unit 410. Optionally, the computing unit can recalculate the target coloration 160 and take into account the changed behavior of the stain device 430 upon detection of a different color application. The recorder 440 may also be used, for example, when the output 170 of the target coloration 160 is given out on a display 322 and a user manually applies the colors to a dental restoration 405, in which case the color application is controlled by the user, in that for example the dental restoration is recorded and the optically detected colors are reported back to the computing unit 310, 410.

It will be apparent to those skilled in the art that the above-described embodiments are to be read by way of example, and that the invention is not limited to them, but that they can be varied in many ways without departing from the scope of the claims.

### List of reference numbers:

- 110: creation of a tooth measurement
- 112: creation of a first tooth measurement
- 114: creation of a second tooth measurement
- 120: providing tooth measurement data
- 122: providing first tooth measurement data
- 124: providing second tooth measurement data
- 130: providing restoration data
- 140: providing information about staining or glazing colors
- 150: generating a color characteristic
- 160: calculating a multicolor target coloration
- 170: issue of a multicolor target coloration
- 172: visualization on a display device
- 210: dental restoration
- 220: color application preset color type "A"
- 222: color application specification color type "A", changed application density
- 224: color application preset color type "B"
- 226: color application specification color type "B", changed application density
- 228: color application preset color type "B"
- 230: color application preset color type "C"
- 300: device
- 305: displayed target color application for a dental restoration
- 307: printed color application for a dental restoration
- 310: computer unit
- 312: memory
- 314: processor
- 320: printer
- 322: display
- 324: write unit
- 400: device
- 405: dental restoration
- 410: computer unit
- 412: memory
- 414: processor
- 424: write unit
- 430: automated staining device
- 432: color or color type
- 434: spray head
- 436: color image
- 440: optical recorder (feedback unit)

## Claims

1. A system comprising a computer program for designing or virtually preparing color customization of dental restorations, wherein the computer program comprises the following instructions:
- providing (110, 120) tooth measurement data, wherein the tooth measurement data comprise at least one insertion area in which the dental restoration is to be inserted;
- providing (130) restoration data for determining at least the blank material and the blank base color, wherein the restoration data contain at least one of the following information: a blank material, a blank base color, information about the color response of the color when certain coloring means comprising staining or glazing colors which could be but are not limited to coloring liquids are applied or wherein the restoration data (130) is obtained by scanning the restoration material, for example after pre-sintering the restoration material by means of a scanner;
- providing (140) information about staining or glazing colors;
- generating (150) a color characteristic from the tooth measurement data or information taking into account the restoration data if the dental measurement data is not suited to generate a color characteristic due to functional or aesthetic reasons;
- calculating (160) a multicolor target coloration from the color characteristic taking into account information about staining or glazing colors, wherein the calculating a multicolor target coloration (160) comprises applying an algorithm for determining a target color characteristic of the restoration, wherein the algorithm takes into account the restoration data and the information on staining or glazing colors, and creating, using the target color characteristic, colors to be used, color gradients and / or color line widths, and/or wherein the calculating a target coloration (160) comprises providing comparative data, applying an algorithm which performs a comparison of the comparative data with the color characteristic and wherein the algorithm performs a transfer in which the target coloration is generated from the data of the color characteristic, for example taking into account tooth structures, and / or determining color gradients that can be generated from the color characteristic and generating the target color characteristic using the color gradients, the comparative data being obtained from tooth measurement data and target colorations of previous dental restorations and/or the comparative data being obtained from data obtained in laboratory experiments, wherein at least one of the algorithms is adapted to calculate color gradients from the color characteristic (150), wherein interpolation means are used to calculate the color gradients, and/or wherein at least one of the algorithms comprises an artificial intelligence which is designed to independently learn on the basis of a test data set to adapt color distributions to reconstructions for evaluating the color situation of the tooth measurement data and therefrom for calculating the color characteristic (150);
- output (170) of the multicolor target coloration for color customization of the dental restoration.

2. The system according to the preceding claim, wherein the tooth measurement data (110) are prepared in the form of a color image, and are preferably recorded with a 3D camera, and/or wherein the tooth measurement data comprises a comparison area, wherein the comparison area is arranged adjacent to and / or opposite to the insertion area and wherein the target coloration (160) is determined from the insertion area and the comparison area.

3. The system according to any one of the preceding claims, wherein the providing tooth measurement data (120) comprises providing (122) first tooth measurement data of an unprepared tooth and providing (124) second tooth measurement data of a prepared tooth, wherein the prepared tooth is prepared for a later treatment.

4. The system according to any one of the preceding claims, wherein prior to providing tooth measurement data (120, 122, 124), the creation (110, 112, 114) of a tooth measurement, preferably as a colored 3D image of an intraoral dental situation, is included.

5. The system according to any one of the preceding claims, wherein areas of the tooth measurement data are identified from the tooth measurement, such as the insertion area, and wherein a subdivision of the areas is carried out, such as a subdivision in tooth structure areas, and / or wherein the tooth measurement data comprise pixels or lattice points and each pixel or lattice point comprises an image color value, the image color values being associated with a respective area of the tooth measurement data.

6. The system according to any one of the preceding claims, wherein the outputting the target coloration (170) includes at least one of the following:
display (172) of the target coloration on a display device,
display (173) of the colors, color gradients and/or color line widths to be used on the display device,
output (176) of the target coloration on a printer, or
output (174) of the target coloration to a data carrier.

7. The system according to any one of the preceding claims, wherein the dental restoration (405) is adapted to close a tooth gap or is prepared to close a plurality of tooth gaps.

8. The system according to any one of the preceding claims, wherein the output of the target coloration (170) comprises color customization of the dental restoration with different staining or glazing colors in such a way that the dental restoration receives the target coloration.

9. The system according to any one of the preceding claims, wherein after the output of the target coloration (170), the finalizing of the dental restoration takes place.

10. The system according to any one of the preceding claims, further comprising
producing the dental restoration from a blank material, and
applying the target coloration on the restoration and
optional sintering or crystallization of the restoration.

11. The system according to any one of the preceding claims, comprising
a computing unit (310, 410),
with a memory (312, 412) for depositing tooth measurement data, wherein the tooth measurement data comprise at least one insertion area in which the dental restoration is to be inserted, furthermore for storing restoration data and information about staining or glazing colors,
with a processor (314, 414) for generating a color characteristic from the tooth measurement data taking into account the restoration data, and for calculating a multicolor target coloration from the color characteristic taking into account the information on staining or glazing colors,
an output device (320, 322, 324, 424, 430) for outputting the multicolor target coloration for color customization of the dental restoration,
a coloring device (430) for applying the multicolor target coloration to the dental restoration.

12. The system according to the preceding claim, further comprising:
a display device (322) for displaying the target coloration, and/or
a printer (320) for outputting the target coloration, and/or
a writing device (324) for outputting the target coloration on a data carrier.

13. The system according to the preceding claim, wherein the coloring device (430) provides a plurality of different coloring solutions (432) and automatically applies the colors to the dental restoration, preferably with a spray head (434).

14. The system according to the preceding claim, wherein the coloring device (430) provides a plurality of 4 to 30 coloring solutions, preferably 7 to 30, more preferably 7 to 20, particularly preferably more than 6 different coloring solutions (432), and/or wherein the coloring device is adapted to provide the coloring solutions in different line widths, and/or wherein the coloring device has a color carrier (436).

15. The system according to one of claims 11 to 14, further comprising an optical recorder (440) for checking the application of stain to the dental restoration (405).

## Patentansprüche

1. System, umfassend ein Computerprogramm zum Gestalten oder virtuellen Vorbereiten einer Farbangleichung von Dentalrestaurationen, wobei das Computerprogramm die folgenden Anweisungen umfasst:
- Bereitstellen (110, 120) von Zahnmessdaten, wobei die Zahnmessdaten mindestens einen Einführbereich umfassen, in den die Dentalrestauration eingeführt wird;
- Bereitstellen (130) von Restaurationsdaten zur Ermittlung von mindestens dem Rohlingmaterial und der Rohlinggrundfarbe, wobei die Restaurationsdaten mindestens eines aus den folgenden Informationen enthalten: ein Rohlingmaterial, eine Rohlinggrundfarbe, Informationen über das Farbverhalten der Farbe, wenn bestimmte Farbgebemittel, die Färbe- oder Glasurfarben umfassen, die Farbgebungsflüssigkeiten sein können, ohne auf diese beschränkt zu sein, aufgebracht werden, oder wobei die Restaurationsdaten (130) durch Scannen des Restaurationsmaterials, zum Beispiel nach dem Vorsintern des Restaurationsmaterials mittels eines Scanners, erhalten werden;
- Bereitstellen (140) von Informationen über die Färbe- oder Glasurfarben;
- Erzeugen (150) einer Farbcharakteristik aus den Zahnmessdaten oder Informationen unter Berücksichtigung der Restaurationsdaten, wenn die Dentalmessdaten sich aus funktionellen oder ästhetischen Gründen nicht zum Erzeugen einer Farbcharakteristik eignen;
- Berechnen (160) einer mehrfarbigen Zielkolorierung aus der Farbcharakteristik unter Berücksichtigung von Informationen über Färbe- oder Glasurfarben, wobei das Berechnen einer mehrfarbigen Zielkolorierung (160) das Anwenden eines Algorithmus zur Ermittlung einer Zielfarbcharakteristik der Restauration umfasst, wobei der Algorithmus die Restaurationsdaten und die Informationen über Färbe- und Glasurfarben berücksichtigt, sowie das Erstellen, mithilfe der Zielfarbcharakteristik, der zu verwendenden Farben, Farbgradienten und/oder Farblinienbreiten, und/oder wobei das Berechnen einer Zielkolorierung (160) das Bereitstellen von Vergleichsdaten umfasst, wobei ein Algorithmus angewendet wird, der einen Vergleich der Vergleichsdaten mit der Farbcharakteristik durchführt, und wobei der Algorithmus eine Übertragung durchführt, bei der die Zielkolorierung aus den Daten der Farbcharakteristik, zum Beispiel unter Berücksichtigung von Zahnstrukturen, erzeugt wird, und/oder Ermitteln von Farbgradienten, die aus der Farbcharakteristik erzeugt werden, und Erzeugen der Zielfarbcharakteristik mithilfe der Farbgradienten, wobei die Vergleichsdaten aus Zahnmessdaten und Zielkolorierungen früherer Dentalrestaurationen erhalten werden und/oder die Vergleichsdaten aus in Laborexperimenten erhaltenen Daten erhalten werden, wobei mindestens einer der Algorithmen dazu angepasst ist, Farbgradienten aus der Farbcharakteristik (150) zu berechnen, wobei eine Interpolationseinrichtung verwendet wird, um die Farbgradienten zu berechnen und/oder wobei mindestens einer der Algorithmen eine künstliche Intelligenz umfasst, die dazu gestaltet ist, auf Basis eines Testdatensatzes selbständig zu lernen, um Farbverteilungen an Rekonstruktionen anzupassen, um die Farbsituation der Zahnmessdaten zu bewerten und daraus die Farbcharakteristik (150) zu berechnen;
- Ausgabe (170) der mehrfarbigen Zielkolorierung für eine Farbangleichung der Dentalrestauration.

2. System nach dem vorstehenden Anspruch, wobei die Zahnmessdaten (110) in der Form eines Farbbildes vorbereitet werden und bevorzugt mit einer 3D-Kamera aufgezeichnet werden, und/oder wobei die Zahnmessdaten einen Vergleichsbereich umfassen, wobei der Vergleichsbereich angrenzend zu und/oder gegenüber dem Einführbereich angeordnet ist und wobei die Zielkolorierung (160) aus dem Einführbereich und dem Vergleichsbereich ermittelt wird.

3. System nach einem der vorstehenden Ansprüche, wobei das Bereitstellen von Zahnmessdaten (120) das Bereitstellen (122) von ersten Zahnmessdaten eines nicht vorbereiteten Zahns und das Bereitstellen (124) von zweiten Zahnmessdaten eines vorbereiteten Zahns umfasst, wobei der vorbereitete Zahn für eine spätere Behandlung vorbereitet wird.

4. System nach einem der vorstehenden Ansprüche, wobei vor dem Bereitstellen von Zahnmessdaten (120, 122, 124) die Erstellung (110, 112, 114) einer Zahnmessung, bevorzugt als farbiges 3D-Bild einer intraoralen Dentalsituation, beinhaltet ist.

5. System nach einem der vorstehenden Ansprüche, wobei Bereiche der Zahnmessdaten aus der Zahnmessung identifiziert werden, wie der Einführbereich, und wobei eine Unterteilung der Bereiche durchgeführt wird, wie eine Unterteilung in Zahnstrukturbereiche, und/oder wobei die Zahnmessdaten Pixel oder Gitterpunkte umfassen und jedes Pixel oder jeder Gitterpunkt einen Bildfarbwert umfasst, wobei die Bildfarbwerte mit einem entsprechenden Bereich der Zahnmessdaten assoziiert sind.

6. System nach einem der vorstehenden Ansprüche, wobei das Ausgeben der Zielkolorierung (170) mindestens eines der Folgenden beinhaltet:
Anzeige (172) der Zielkolorierung auf einem Anzeigegerät,
Anzeige (173) der zu verwendenden Farben, Farbgradienten und/oder Farblinienbreiten auf dem Anzeigegerät,
Ausgabe (176) der Zielkolorierung auf einem Drucker, oder
Ausgabe (174) der Zielkolorierung an einen Datenträger.

7. System nach einem der vorstehenden Ansprüche, wobei die Dentalrestauration (405) dazu angepasst ist, eine Zahnlücke zu schließen, oder dazu vorbereitet ist, eine Vielzahl von Zahnlücken zu schließen.

8. System nach einem der vorstehenden Ansprüche, wobei die Ausgabe der Zielkolorierung (170) mindestens eine Farbangleichung der Dentalrestauration mit verschiedenen Färbe- oder Glasurfarben in einer Weise umfasst, dass die Dentalrestauration die Zielkolorierung annimmt.

9. System nach einem der vorstehenden Ansprüche, wobei nach dem Ausgeben der Zielkolorierung (170) die Fertigstellung der Dentalrestauration erfolgt.

10. System nach einem der vorstehenden Ansprüche, ferner umfassend
Herstellen der Dentalrestauration aus einem Rohlingmaterial, und
Anwenden der Zielkolorierung auf die Restauration und
optional Sintern oder Kristallisieren der Restauration.

11. System nach einem der vorstehenden Ansprüche, umfassend
eine Recheneinheit (310, 410),
mit einem Speicher (312, 412) zum Hinterlegen von Zahnmessdaten, wobei die Zahnmessdaten mindestens einen Einführbereich umfassen, in den die Dentalrestauration eingeführt werden soll, weiterhin zum Speichern von Restaurationsdaten und Informationen über Färbe- oder Glasurfarben,
mit einem Prozessor (314, 414) zum Erzeugen einer Farbcharakteristik aus den Zahnmessdaten unter Berücksichtigung der Restaurationsdaten, sowie zum Berechnen einer mehrfarbigen Zielkolorierung aus der Zahncharakteristik unter Berücksichtigung der Informationen über Färbe- und Glasurfarben,
eine Ausgabevorrichtung (320, 322, 324, 424, 430) zum Ausgeben der mehrfarbigen Zielkolorierung für die Farbangleichung der Dentalrestauration,
eine Farbgebungsvorrichtung (430) zum Anwenden der mehrfarbigen Zielkolorierung auf die Zahnrestauration.

12. System nach dem vorstehenden Anspruch, ferner umfassend:
eine Anzeigevorrichtung (322) zum Anzeigen der Zielkolorierung, und/oder
einen Drucker (320) zum Ausgeben der Zielkolorierung, und/oder
eine Schreibvorrichtung (324) zum Ausgeben der Zielkolorierung auf einem Datenträger.

13. System nach dem vorstehenden Anspruch, wobei die Farbgebungsvorrichtung (430) eine Vielzahl von verschiedenen Farbgebungslösungen (432) bereitstellt und die Farben automatisch, bevorzugt mit einem Sprühkopf (434), auf die Zahnrestauration anwendet.

14. System nach dem vorstehenden Anspruch, wobei die Farbgebungsvorrichtung (430) eine Vielzahl von 4 bis 30 Farbgebungslösungen, bevorzugt 7 bis 30, mehr bevorzugt 7 bis 20, besonders bevorzugt mehr als 6 verschiedene Farbgebungslösungen (432) bereitstellt, und/oder wobei die Farbgebungsvorrichtung dazu angepasst ist, die Farbgebungslösungen in unterschiedlichen Linienbreiten bereitzustellen, und/oder wobei die Farbgebungsvorrichtung einen Farbträger (436) aufweist.

15. System nach einem der Ansprüche 11 bis 14, ferner umfassend ein optisches Aufzeichnungsgerät (440) zum Prüfen der Anwendung von Färbung auf die Dentalrestauration (405).

## Revendications

1. Système comprenant un programme informatique pour concevoir ou préparer virtuellement une personnalisation des couleurs de restaurations dentaires, dans lequel le programme informatique comprend les instructions suivantes :
- la fourniture (110, 120) de données de mesure de dent, dans lequel les données de mesure de dent comprennent au moins une zone d'insertion dans laquelle la restauration dentaire doit être insérée ;
- la fourniture (130) de données de restauration pour déterminer au moins le matériau d'ébauche et la couleur de base d'ébauche, dans lequel les données de restauration contiennent au moins l'une des informations suivantes : un matériau d'ébauche, une couleur de base d'ébauche, des informations concernant la réponse chromatique de la couleur lorsque sont appliqués certains moyens de coloration comprenant des couleurs de coloration ou d'émaillage qui pourraient être, mais sans s'y limiter, des liquides colorants ou dans lequel les données de restauration (130) sont obtenues par balayage du matériau de restauration, par exemple après pré-frittage du matériau de restauration au moyen d'un scanner ;
- la fourniture (140) d'informations concernant les couleurs de coloration ou d'émaillage ;
- la génération (150) d'une caractéristique de couleur à partir des données de mesure de dent ou d'informations tenant compte des données de restauration si les données de mesure dentaire ne sont pas adaptées pour générer une caractéristique de couleur pour des raisons fonctionnelles ou esthétiques ;
- le calcul (160) d'une coloration cible multicolore à partir de la caractéristique de couleur en tenant compte des informations concernant les couleurs de coloration ou d'émaillage, dans lequel le calcul d'une coloration cible multicolore (160) comprend l'application d'un algorithme pour déterminer une caractéristique de couleur cible de la restauration, dans lequel l'algorithme tient compte des données de restauration et des informations sur les couleurs de coloration ou d'émaillage, et la création, à l'aide de la caractéristique de couleur cible, de couleurs à utiliser, de dégradés de couleurs et/ou de largeurs de trait de couleur, et/ou dans lequel le calcul d'une coloration cible (160) comprend la fourniture de données comparatives, l'application d'un algorithme qui réalise une comparaison des données comparatives avec la caractéristique de couleur et dans lequel l'algorithme réalise un transfert dans lequel la coloration cible est générée à partir des données de la caractéristique de couleur, par exemple en tenant compte des structures de dent, et/ou la détermination de dégradés de couleurs qui peuvent être générés à partir de la caractéristique de couleur et la génération de la caractéristique de couleur cible à l'aide des dégradés de couleurs, les données comparatives étant obtenues à partir de données de mesure de dent et de colorations cibles de restaurations dentaires précédentes et/ou les données comparatives étant obtenues à partir de données obtenues lors d'expériences en laboratoire, dans lequel au moins un des algorithmes est adapté pour calculer des dégradés de couleurs à partir de la caractéristique de couleur (150), dans lequel des moyens d'interpolation sont utilisés pour calculer les dégradés de couleurs, et/ou dans lequel au moins un des algorithmes comprend une intelligence artificielle qui est conçue pour apprendre de manière indépendante, sur la base d'un ensemble de données d'essai, à adapter des distributions de couleurs à des reconstructions pour évaluer la situation de couleur des données de mesure de dent et, à partir de là, pour calculer la caractéristique de couleur (150) ;
- la production en sortie (170) de la coloration cible multicolore pour une personnalisation des couleurs de la restauration dentaire.

2. Système selon la revendication précédente, dans lequel les données de mesure de dent (110) sont préparées sous la forme d'une image couleur, et sont de préférence enregistrées avec une caméra 3D, et/ou dans lequel les données de mesure de dent comprennent une zone de comparaison, dans lequel le la zone de comparaison est agencée de manière adjacente et/ou opposée à la zone d'insertion et dans lequel la coloration cible (160) est déterminée à partir de la zone d'insertion et de la zone de comparaison.

3. Système selon l'une quelconque des revendications précédentes, dans lequel la fourniture de données de mesure de dent (120) comprend la fourniture (122) de premières données de mesure de dent d'une dent non préparée et la fourniture (124) de secondes données de mesure de dent d'une dent préparée, dans lequel la dent préparée est préparée pour un traitement ultérieur.

4. Système selon l'une quelconque des revendications précédentes, dans lequel avant la fourniture des données de mesure de dent (120, 122, 124), est incluse la création (110, 112, 114) d'une mesure de dent, de préférence sous la forme d'une image 3D en couleur d'une situation dentaire intrabuccale.

5. Système selon l'une quelconque des revendications précédentes, dans lequel des zones des données de mesure de dent sont identifiées à partir de la mesure de dent, telles que la zone d'insertion, et dans lequel une subdivision des zones est effectuée, telle qu'une subdivision en zones de structure de dent, et/ou dans lequel les données de mesure de dent comprennent des pixels ou des points de réseau et chaque pixel ou point de réseau comprend une valeur de couleur d'image, les valeurs de couleur d'image étant associées à une zone respective des données de mesure de dent.

6. Système selon l'une quelconque des revendications précédentes, dans lequel la production en sortie de la coloration cible (170) inclut au moins l'un des suivants :
l'affichage (172) de la coloration cible sur un dispositif d'affichage,
l'affichage (173) des couleurs, dégradés de couleurs et/ou largeurs de trait de couleur à utiliser sur le dispositif d'affichage,
la production en sortie (176) de la coloration cible sur une imprimante, ou
la production en sortie (174) de la coloration cible vers un support de données.

7. Système selon l'une quelconque des revendications précédentes, dans lequel la restauration dentaire (405) est adaptée pour fermer un espace de dent ou est préparée pour fermer une pluralité d'espaces de dent.

8. Système selon l'une quelconque des revendications précédentes, dans lequel la production en sortie de la coloration cible (170) comprend une personnalisation des couleurs de la restauration dentaire avec différentes couleurs de coloration ou d'émaillage de telle sorte que la restauration dentaire reçoive la coloration cible.

9. Système selon l'une quelconque des revendications précédentes, dans lequel après la production en sortie de la coloration cible (170), a lieu la finalisation de la restauration dentaire.

10. Système selon l'une quelconque des revendications précédentes, comprenant en outre
la production de la restauration dentaire à partir d'un matériau d'ébauche, et
l'application de la coloration cible sur la restauration et
le frittage ou la cristallisation éventuel(le) de la restauration.

11. Système selon l'une quelconque des revendications précédentes, comprenant
une unité informatique (310, 410),
avec une mémoire (312, 412) pour consigner des données de mesure de dent, dans laquelle les données de mesure de dent comprennent au moins une zone d'insertion dans laquelle la restauration dentaire doit être insérée, pour stocker en outre des données de restauration et des informations concernant les couleurs de coloration ou d'émaillage,
avec un processeur (314, 414) pour générer une caractéristique de couleur à partir des données de mesure de dent en tenant compte des données de restauration, et pour calculer une coloration cible multicolore à partir de la caractéristique de couleur en tenant compte des informations sur les couleurs de coloration ou d'émaillage,
un dispositif de sortie (320, 322, 324, 424, 430) pour produire en sortie la coloration cible multicolore pour une personnalisation des couleurs de la restauration dentaire,
un dispositif de coloration (430) pour appliquer la coloration cible multicolore à la restauration dentaire.

12. Système selon la revendication précédente, comprenant en outre :
un dispositif d'affichage (322) pour afficher la coloration cible, et/ou
une imprimante (320) pour produire en sortie la coloration cible, et/ou
un dispositif d'écriture (324) pour produire en sortie la coloration cible sur un support de données.

13. Système selon la revendication précédente, dans lequel le dispositif de coloration (430) fournit une pluralité de solutions colorantes (432) différentes et applique automatiquement les couleurs à la restauration dentaire, de préférence avec une tête de pulvérisation (434).

14. Système selon la revendication précédente, dans lequel le dispositif de coloration (430) fournit une pluralité de 4 à 30 solutions colorantes, de préférence de 7 à 30, plus préférablement de 7 à 20, de manière particulièrement préférée plus de 6 solutions colorantes (432) différentes, et/ou dans lequel le dispositif de coloration est adapté pour fournir les solutions de coloration dans différentes largeurs de trait, et/ou dans lequel le dispositif de coloration présente un support de couleurs (436).

15. Système selon l'une des revendications 11 à 14, comprenant en outre un enregistreur optique (440) pour vérifier l'application de colorant à la restauration dentaire (405).
